# EUROPÄISCHE PATENTSCHRIFT

(11) **EP 4 243 917 B1**
(45) Veröffentlichungstag und Bekanntmachung des Hinweises auf die Patenterteilung: **07.08.2024**
(21) Anmeldenummer: 21802243.2
(22) Anmeldetag: 27.10.2021
(51) Int. Cl.: A61M 37/00, A61K 9/00

(54) **INTRADERMALES WIRKSTOFFABGABESYSTEM**
INTRADERMAL ACTIVE INGREDIENT DELIVERY SYSTEM
SYSTÈME D'ADMINISTRATION INTRADERMIQUE D'UN PRINCIPE ACTIF

(30) Priorität: 12.11.2020 DE 102020129916
(43) Veröffentlichungstag der Anmeldung: 20.09.2023
(73) Patentinhaber: LTS Lohmann Therapie-Systeme AG, 56626 Andernach (DE)
(72) Erfinder: SCHOMISCH, Sebastian, 56727 Mayen (DE)
(74) Vertreter: dompatent von Kreisler Selting Werner - Partnerschaft von Patent- und Rechtsanwälten mbB
(86) Internationale Anmeldenummer: PCT/EP2021/079871
(87) Internationale Veröffentlichungsnummer: WO 2022/101017

(56) Entgegenhaltungen:
- EP-A1- 3 338 853
- US-A1- 2016 296 149
- US-B1- 10 105 080
- US-B2- 8 920 375

## Beschreibung

Die Erfindung betrifft ein intradermales Wirkstoffabgabesystem, mit dem ein insbesondere medizinischer Wirkstoff intradermal an einen Patienten abgegeben werden kann.

Zur Wirkstoffabgabe sind transdermale therapeutische Systeme (TTS) bekannt. Beispielsweise können transdermale Pflaster auf die Haut des Patienten geklebt werden. Der gespeicherte Wirkstoff wird kontinuierlich über die Haut resorbiert. Diese Verabreichungsform hat den Vorteil, dass sie im Unterschied zur Injektion für den Patienten schmerzfrei ist und ferner eine Medikation über einen längeren Zeitraum erfolgen kann. Nachteilig ist allerdings, dass eine Anpassung der Medikation nur dadurch erfolgen kann, dass das TTS entfernt oder ersetzt wird. Es ist insbesondere schwierig, die an den Patienten abgegebene Menge eindeutig zu bestimmen.

Ferner ist es bekannt, über Mikronadeln Wirkstoffe zu verabreichen. Hierbei wird der Wirkstoff über eine Vielzahl von Nadeln an die Dermis der Haut abgegeben. Die Abgabe erfolgt somit in eine äußere Schicht der Haut, in der keine Nerven angeordnet sind, so dass auch diese Wirkstoffabgabe für den Patienten schmerzfrei ist. Hierbei sind Nadeln bekannt, die als Hohlnadel ausgebildet sind, in denen Wirkstoff enthalten ist und durch die Nadelöffnung an der Nadelspitze abgegeben wird. Ferner sind Mikronadeln bekannt, die aus einem Material bestehen, das sich in der Haut auflöst. Hierbei ist der Wirkstoff in das Nadelmaterial eingebracht. Auch hierbei besteht die Problematik, dass eine genaue Bestimmung der an den Patienten abgegebenen Wirkstoffmenge schwierig ist, da sich beispielsweise die Nadeln nicht vollständig auflösen, abbrechen können oder dergleichen.

Aufgabe der Erfindung ist es, ein intradermales Wirkstoffabgabesystem zu schaffen, mit dem die an den Patienten abgegebene Wirkstoffmenge besser bestimmbar ist.

Die Lösung der Aufgabe folgt erfindungsgemäß durch ein intradermales Wirkstoffabgabesystem mit den Merkmalen des Anspruchs 1.

Das erfindungsgemäße intradermale Wirkstoffabgabesystem weist eine Entnahmeeinrichtung zur Entnahme von Interstitial-Flüssigkeit auf. Vorzugsweise weist die Entnahmeeinrichtung insbesondere mehrere als Hohlnadeln ausgebildete Mikronadeln auf. Mit der Entnahmeeinrichtung ist eine Fördereinrichtung fluidisch verbunden. Mit Hilfe der Fördereinrichtung erfolgt insbesondere ein Ansaugen der Interstitial-Flüssigkeit aus dem Gewebe des Patienten. Ferner weist das erfindungsgemäße intradermale Wirkstoffabgabesystem ein Wirkstoffreservoir auf. Das Wirkstoffreservoir ist mit der Fördereinrichtung fluidisch verbunden. In dem Wirkstoffreservoir ist der Wirkstoff, der dem Patienten verabreicht werden soll, in fester und/oder pulverförmiger und/oder flüssiger Form bereitgestellt. Erfindungsgemäß ist mit der Fördereinrichtung ferner fluidisch eine Abgabeeinrichtung verbunden. Über die Abgabeeinrichtung erfolgt eine intradermale Abgabe eines Gemisches aus Wirkstoff und entnommener Interstitial-Flüssigkeit bzw. eine Abgabe der Interstitial-Flüssigkeit, die mit Wirkstoff angereichert ist, an den Patienten. Die Abgabeeinrichtung weist vorzugsweise insbesondere als Hohlnadeln ausgebildete Mikronadeln auf.

Mithilfe des erfindungsgemäßen intradermalen Wirkstoffabgabesystems ist es somit möglich, dem Patienten eine klar definierte Wirkstoffmenge zu verabreichen. Hierbei ist es bevorzugt, dass in dem Wirkstoffreservoir der Wirkstoff in konzentrierter Form vorliegt und durch die Interstitial-Flüssigkeit verdünnt wird bzw. durch die Interstitial-Flüssigkeit gelöst wird.

In besonders bevorzugter Weiterbildung der Erfindung weist die Entnahmeeinrichtung insbesondere eine Vielzahl von Mikronadeln auf. Vorzugsweise ist eines oder mehrere Mikronadelarrays vorgesehen, die in bevorzugter Ausführungsform mindestens 4 Nadeln/cm², vorzugsweise mindestens 50 Nadeln/cm² und besonders bevorzugt mindestens 100 Nadeln/cm², je Array aufweisen.

Insbesondere wenn die Entnahmeeinrichtung mehrere Mikronadelarrays aufweist, ist es bevorzugt, dass diese zueinander parallel angeordnet sind und jeweils über einen gesonderten Kanal mit der Fördereinrichtung verbunden sind. Ferner ist es möglich, dass mehrere Entnahmeeinrichtungen in gleichem Abstand zur Fördereinrichtung, insbesondere kreisförmig, um die Fördereinrichtung herum angeordnet sind. Das Vorsehen mehrerer Mikronadelarrays hat insbesondere den Vorteil, dass diese jeweils mit Kanälen mit geringem Querschnitt verbunden werden können und insofern ein sehr kleines intradermales Wirkstoffsystem realisiert werden kann. Das Vorsehen mehrerer Mikronadelarrays hat ferner den Vorteil, dass stets eine ausreichende Flüssigkeitsversorgung sichergestellt ist. Die ansaugbare Flüssigkeit kann insbesondere in Verbindung mit einer Flüssigkeitsfördereinrichtung mit einer Pumpe mit erhöhtem Hub und/oder erhöhter Hubzahl realisiert werden.

Vorzugsweise weist die Fördereinrichtung mindestens eine Pumpe auf. Bei der Pumpe kann es sich um eine Membranpumpe oder dergleichen handeln, die insbesondere derart ausgestaltete Ventile aufweist, dass eine Flüssigkeitsförderung nur in eine Richtung möglich ist. Insbesondere sind Membrane jeweils am Einlass und am Auslass der Pumpe vorgesehen. Hierdurch ist insbesondere sichergestellt, dass das Mischen mit dem Wirkstoff vorzugsweise erst innerhalb der Pumpe bzw. einem innerhalb der Pumpe vorgesehenen Raum stattfindet. Es ist insbesondere vermieden, dass sich der Wirkstoff nicht bereits ohne aktives Pumpen oder Dosieren verdünnt. Des Weiteren weist das Vorsehen von Membranen an den Mikronadelarrays den Vorteil auf, dass die Kanäle stets konstant mit Flüssigkeit bzw. eine Wirkstofflösung gefüllt sind und ein Rückfluss in die Haut oder eine in der Pumpe angeordnete Kammer vermieden ist. Die Steuerung der Pumpe könnte beispielsweise über einen Piezoaktivator erfolgen, der zur Aktivierung des Pumpvorgangs eine Druckveränderung auf eine Pumpenkammer bewirkt. Denkbar ist auch, dass das Pumpen durch manuell erzeugten Druck auf eine entsprechende Kammer erfolgt.

Bei einer besonders bevorzugten Ausführungsform der Erfindung ist ein flüssigen Wirkstoff enthaltendes Wirkstoffreservoir mit einem Einlass der Fördereinrichtung verbunden. Vorzugsweise ist auch die Entnahmeeinrichtung mit dem Einlass der Fördereinrichtung verbunden. Insbesondere erfolgt die Verbindung mit dem Einlass einer Pumpe. Die Pumpe kann einen oder mehrere Einlässe aufweisen, wobei zur Miniaturisierung des Wirkstoffabgabesystems das Vorsehen eines einzigen Einlasses bevorzugt ist.

Bei dieser bevorzugten Ausführungsform der Erfindung kann durch die Fördereinrichtung bereits gleichzeitig flüssiger Wirkstoff und Interstitial-Flüssigkeit angesaugt werden. Hierbei erfolgt vorzugsweise ein Mischen der beiden Flüssigkeiten. Gegebenenfalls kann insbesondere innerhalb der Fördereinrichtung eine Mischkammer vorgesehen sein, in der ein Durchmischen der beiden Flüssigkeiten erfolgt. Bei der Kammer kann es sich insbesondere um eine Kammer der Pumpe, insbesondere der Membranpumpe, handeln. Die Abgabe der beiden gemischten Flüssigkeiten erfolgt sodann vorzugsweise unter Zuhilfenahme der Fördereinrichtung an die Dermis der Haut über eine mit der Fördereinrichtung verbundene Abgabeeinrichtung.

Bei einer weiteren bevorzugten Ausführungsform ist das Wirkstoffreservoir mit einem Auslass der Fördereinrichtung, insbesondere einem Auslass einer Pumpe, der Fördereinrichtung verbunden. Hierbei kann in dem Wirkstoffreservoir ein flüssiger, fester oder pulverförmiger Wirkstoff vorgesehen sein. Mithilfe der Fördereinrichtung erfolgt bei dieser bevorzugten Ausführungsform vorzugsweise ein Ansaugen der Interstitial-Flüssigkeit über die Entnahmeeinrichtung und ein Fördern der Interstitial-Flüssigkeit in das Wirkstoffreservoir. Bei dieser Ausführungsform kann das Wirkstoffreservoir gleichzeitig als Mischkammer dienen bzw. es erfolgt ein Mischen oder Lösen des Wirkstoffs in dem Wirkstoffreservoir.

Die Fördereinrichtung kann in bevorzugter Ausführungsform mehrere, insbesondere zwei Pumpen aufweisen. Bei dieser Ausführungsform ist es möglich, dass das Wirkstoffreservoir zwischen den beiden Pumpen angeordnet ist. Die in Strömungsrichtung vor dem Wirkstoffreservoir angeordnete Pumpe dient hiermit zum Ansaugen der Interstitial-Flüssigkeit und anschließenden Pumpen der Interstitial-Flüssigkeit in das Wirkstoffreservoir. Die in Strömungsrichtung hinter dem Reservoir angeordnete Pumpe saugt die mit Wirkstoff angereicherte Interstitial-Flüssigkeit an und fördert diese zur Abgabeeinrichtung.

Die insbesondere eine Vielzahl von Hohlnadeln aufweisende Abgabeeinrichtung ist insbesondere über einen oder mehrere Kanäle in bevorzugter Ausführungsform mittelbar oder unmittelbar mit der Fördereinrichtung verbunden.

Bei einer weiteren bevorzugten Ausführungsform kann die Abgabeeinrichtung insbesondere über Kanäle mittelbar oder unmittelbar mit der Wirkstoffkammer oder einer Mischkammer verbunden sein. Bei dieser Ausführungsform ist es bevorzugt, dass die Pumpe angesaugte Interstitial-Flüssigkeit durch das Wirkstoffreservoir und die Mischkammer hindurch fördert. Hierbei nimmt die Interstitial-Flüssigkeit Wirkstoff auf, so dass mit Wirkstoff angereicherte Interstitial-Flüssigkeit zur Abgabeeinrichtung gelangt.

Bei einer weiteren bevorzugten Ausführungsform des erfindungsgemäßen intradermalen Wirkstoffabgabesystem ist das Wirkstoffreservoir oder ein zusätzliches Wirkstoffreservoir in die Abgabeeinrichtung integriert. Das Mischen der Interstitial-Flüssigkeit mit dem in einem derartigen Wirkstoffreservoir angeordneten Wirkstoff erfolgt somit unmittelbar vor der Abgabe an den Patienten. Insbesondere kann der Wirkstoff unmittelbar in den Hohlnadeln angeordnet sein.

Gegebenenfalls kann das Wirkstoffsystem eine Mischkammer aufweisen. Hierbei kann auf die Mischkammer ein Mischelement wie ein Piezoaktuator oder dergleichen einwirken, um ein gutes Durchmischen des Wirkstoffs mit der Interstitial-Flüssigkeit zu bewirken. Auch kann hierbei bei einem Wirkstoff in fester oder pulverförmiger Form ein zuverlässiges Lösen des Wirkstoffs gewährleistet werden. Die Mischkammer kann je nach Ausführungsform entfallen oder beispielsweise in die Fördereinrichtung das Wirkstoffreservoir und/oder die Abgabeeinrichtung integriert sein.

Das erfindungsgemäße Wirkstoffabgabesystem weist eine Größe auf, so dass das gesamte System auf einfache Weise zuverlässig auf der Haut des Patienten angeordnet werden kann. Neben der kleinen Ausgestaltung werden auf einer Berührungsfläche mit der Haut von nur wenigen cm² könnten auch Wirkstoffabgabesysteme realisiert werden, die beispielsweise in Form eines Armbands den Arm umschließen. Besonders wichtig zur Realisierung des erfindungsgemäßen Wirkstoffabgabesystems ist die Flexibilität des Systems und die Eindringtiefe der Nadeln. Zur Realisierung der erforderlichen Flexibilität ist das System stets so dünn ausgestaltet, dass es dauerhaft auch bei Bewegungen mit der gesamten Fläche der Haut in Kontakt bleibt und andererseits die Bewegungsfreiheit des Patienten nur geringfügig eingeschränkt ist. Die Nadeln weisen eine Länge auf, dass diese nur in die Dermis der Haut eindringen, so dass ein ggf. schmerzhafter Kontakt mit Nerven vermieden ist.

Nachfolgend wird die Erfindung anhand bevorzugter Ausführungsformen unter Bezugnahme auf die anliegenden Zeichnungen näher erläutert.

Es zeigen:
- Figuren 1 - 4: unterschiedliche bevorzugte Ausführungsformen des erfindungsgemäßen intradermalen Wirkstoffabgabesystems,
- Figur 5: eine schematische stark vereinfachte Schnittansicht einer Entnahmeeinrichtung oder einer Abgabeeinrichtung und
- Figur 6: eine schematische stark vereinfachte Darstellung einer Fördereinrichtung in Form einer Membranpumpe.

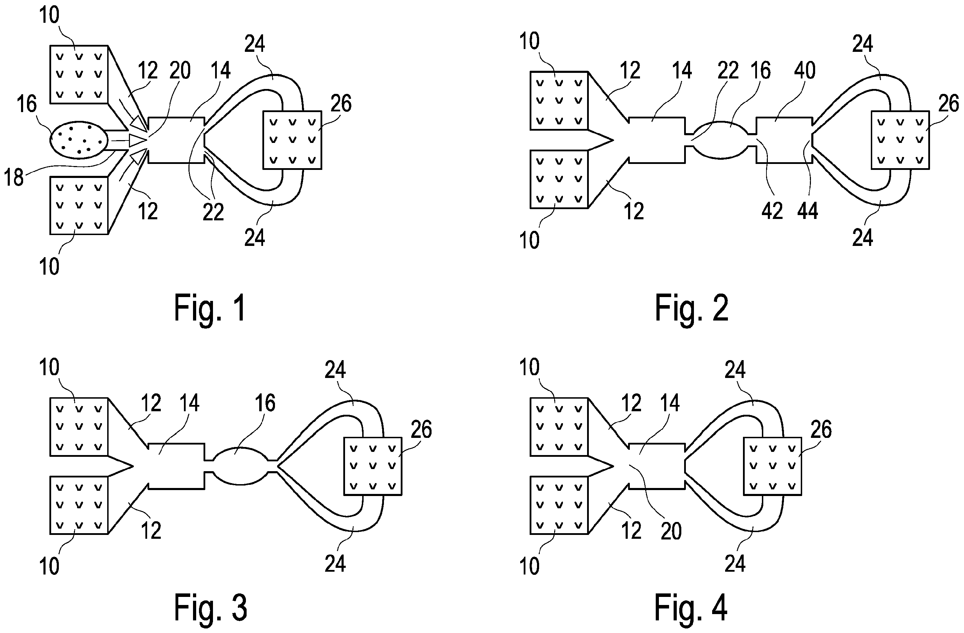

In den unterschiedlichen in den Figuren 1 - 4 dargestellten Ausführungsformen des erfindungsgemäßen intradermalen Wirkstoffabgabesystems sind identische und ähnliche Bauteile mit denselben Bezugszeichen gekennzeichnet.

Die in Figur 1 dargestellte Ausführungsform weist eine Entnahmeeinrichtung 10 auf, die zwei Mikroarrays mit einer Vielzahl von Hohlnadeln umfasst. Die beiden Mikroarrays 10 sind über Kanäle 12 jeweils mit einer Pumpe bzw. einer Fördereinrichtung 14 verbunden. Ein Wirkstoffreservoir 16, in dem flüssiger Wirkstoff bevorratet ist, ist über einen Kanal 18 ebenfalls mit der Pumpe 14 verbunden. Im dargestellten Ausführungsbeispiel sind alle drei Kanäle 12,18 vor der Pumpe 14 zusammengeführt und mit einem einzigen Pumpeneinlass 20 verbunden.

Die Pumpe 14 weist im dargestellten Ausführungsbeispiel zwei Pumpenauslässe 22 auf, die jeweils mit einem Kanal 24 verbunden sind. Alternativ kann die Pumpe 14 auch einen Auslass aufweisen, der sodann ebenfalls mit zwei Kanälen 24 verbunden ist. Die Kanäle 24 sind mit einer Abgabeeinrichtung 26 verbunden. Bei der Abgabeeinrichtung 26 handelt es sich um ein Mikroarray mit einer Vielzahl von Hohlnadeln.

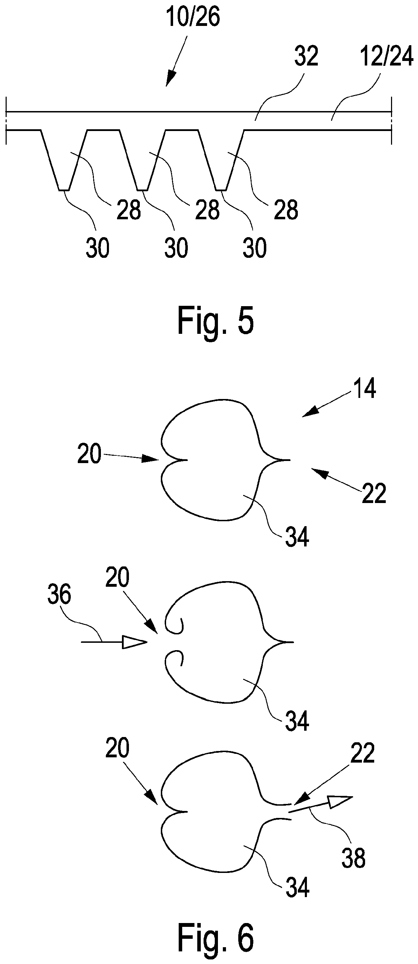

Figur 5 zeigt schematisch einen Ausschnitt eines Mikroarrays, wobei es sich um ein Mikroarray 10 der Entnahmeeinrichtung oder ein Mikroarray 26 der Abgabeeinrichtung handeln kann. Das Mikroarray 10,26 weist eine Vielzahl von Hohlnadeln 28 auf, die an der Spitze eine Öffnung 30 aufweisen. Durch die Öffnung 30 kann, wenn es sich um eine Entnahmeeinrichtung handelt, Interstitial-Flüssigkeit angesaugt werden. Wenn es sich bei dem Mikroarray um eine Abgabeeinrichtung handelt, wird durch die Öffnungen 30 die mit Wirkstoff versetzte Interstitial-Flüssigkeit an den Patienten abgegeben. Die Hohlnadeln 28 sind auf der den Öffnungen 30 gegenüberliegenden Seite mit einem Kanal 32 verbunden. Der Kanal 32 erstreckt sich vorzugsweise über die gesamte Oberfläche des Mikroarrays und ist entweder mit dem Kanal 12 oder mit dem Kanal 24 verbunden.

Mit Hilfe des in Figur 1 dargestellten intradermalen Wirkstoffabgabesystems kann von der Pumpe über die Hohlnadeln der Mikroarrays 10 Interstitial-Flüssigkeit durch die Kanäle 12 angesaugt werden. Gleichzeitig wird aus dem Wirkstoffreservoir 16 flüssiger Wirkstoff angesaugt. Innerhalb der Pumpe 14 bzw. bereits in dem Bereich, in dem die Kanäle 12 und 18 zusammengeführt sind, erfolgt ein Durchmischen der beiden Flüssigkeiten. Nach Verschließen des Pumpeneinlasses 20 kann mit Hilfe der Pumpe 14 das Flüssigkeitsgemisch durch die beiden Auslässe 22 und die Kanäle 24 zu den Mikronadeln 30 des Abgabesystems 26 gefördert und dem Patienten injiziert werden.

Als Pumpe 14 kann beispielsweise eine schematisch in Figur 6 dargestellte Membranpumpe vorgesehen sein. In dem in Figur 6 dargestellten Zustand der Pumpe 14 ist sowohl der Auslass 22 als auch der Einlass 20 geschlossen. Die Pumpe 14 befindet sich in unbelastetem Zustand. Beispielsweise durch ein nicht dargestelltes Piezoelement kann auf einen Pumpenhohlraum 34 Kraft ausgeübt werden. Bei einer Erzeugung von Unterdruck oder Vakuum in dem Pumpenhohlraum 34 öffnet sich der Einlass 20, so dass Flüssigkeit in Richtung eines Pfeils 36 in den Hohlraum 34 einströmen kann. Zum Fördern der Flüssigkeit aus dem Pumpenhohlraum 34 wird wieder, beispielsweise mittels eines Piezoelements, Druck auf die Hohlraum 34 ausgeübt. Hierdurch verschließt sich der Einlass 20 entsprechend der unteren Darstellung in Figur 6. Gleichzeitig öffnet sich der Auslass 22, so dass Flüssigkeit in Richtung eines Pfeils 38 strömt.

Gemäß einer weiteren in Figur 2 dargestellten Ausführungsform sind die beiden Mikroarrays 10 der Entnahmeeinrichtung wiederum über Kanäle 12 mit einer ersten Pumpe 14 verbunden. In diesem Ausführungsbeispiel ist der Auslass 22 der ersten Pumpe 14 mit dem Wirkstoffreservoir 16 verbunden. Das Wirkstoffreservoir 16 ist dann in Strömungsrichtung, d.h. in Figur 2 nach rechts, mit einer zweiten Pumpe 40 über deren Einlass 42 verbunden. Ein Auslass 44 der zweiten Pumpe 40 ist über Kanäle 24 mit der Abgabeeinrichtung 26 verbunden.

Bei dem in der Figur 2 dargestellten Ausführungsbeispiel saugt die erste Pumpe 14 somit über die Entnahmeeinrichtung 10 Interstitial-Flüssigkeit an und fördert diese in das Wirkstoffreservoir 16. In dem Wirkstoffreservoir 16 ist ein flüssiger, fester oder pulverförmiger Wirkstoff vorgesehen. Das Wirkstoffreservoir 16 dient somit in dem in Fig. 2 dargestellten Ausführungsbeispiel gleichzeitig als Mischkammer. Die zweite Pumpe 40 saugt aus dem Wirkstoffreservoir 16 das Flüssigkeitsgemisch an und fördert dieses über die Kanäle 24 zur Abgabeeinrichtung 26.

Das in Fig. 3 dargestellte Ausführungsbeispiel entspricht im Wesentlichen dem in Figur 2 dargestellten Ausführungsbeispiel, mit dem Unterschied, dass keine zweite Pumpe vorgesehen ist. Die von der Pumpe 14 angesaugte Interstitial-Flüssigkeit wird somit von der Pumpe 14 durch das Wirkstoffreservoir 16 hindurchgepumpt, wobei die Interstitial-Flüssigkeit Wirkstoff aufnimmt. Die Pumpe 14 fördert das Flüssigkeitsgemisch sodann unmittelbar durch die Kanäle 24 weiter zu der Abgabeeinrichtung 26.

Die in Figur 4 dargestellte Ausführungsform entspricht im Wesentlichen der in Figur 1 dargestellten Ausführungsform, wobei mit dem Pumpeneinlass 20 kein Wirkstoffreservoir 16 verbunden ist. Vielmehr ist bei diesem Ausführungsbeispiel das Wirkstoffreservoir in die Entnahmeeinrichtung 26 integriert. Beispielsweise ist der Wirkstoff in den Hohlnadeln 30 und/oder in dem Kanalbereich 32 (Figur 5) vorgesehen und wird von der Interstitial-Flüssigkeit unmittelbar vor dem Injizieren aufgenommen.

## Patentansprüche

1. Intradermales Wirkstoffabgabesystem, mit
einer Entnahmeeinrichtung (10) zur Entnahme von Interstitial-Flüssigkeit,
einer mit der Entnahmeeinrichtung (10) fluidisch verbundenen Fördereinrichtung (14) zum Fördern von Flüssigkeit,
einem mit der Fördereinrichtung fluidisch verbundenen Wirkstoffreservoir (16) und
einer mit der Fördereinrichtung (14) fluidisch verbundenen Abgabeeinrichtung (26) zur intradermalen Abgabe der Wirkstoff aufweisenden Interstitial-Flüssigkeit.

2. Intradermales Wirkstoffabgabesystem nach Anspruch 1, **dadurch gekennzeichnet, dass** die Entnahmeeinrichtung (10) Mikronadeln (30), insbesondere ein Mikroarray, mit mindestens 4 Nadeln/cm², insbesondere mindestens 50 Nadeln/cm² und besonders bevorzugt mindestens 100 Nadeln/cm², aufweist.

3. Intradermales Wirkstoffabgabesystem nach Anspruch 2, **dadurch gekennzeichnet, dass** die Entnahmeeinrichtung (10) zwei Mikroarrays mit jeweils insbesondere mindestens 4 Nadeln/cm², insbesondere mindestens 50 Nadeln/cm² und besonders bevorzugt mindestens 100 Nadeln/cm², aufweist.

4. Intradermales Wirkstoffabgabesystem nach einem der Ansprüche 1-3, **dadurch gekennzeichnet, dass** die Fördereinrichtung (16) mindestens eine Pumpe aufweist.

5. Intradermales Wirkstoffabgabesystem nach einem der Ansprüche 1-4, **dadurch gekennzeichnet, dass** in dem Wirkstoffreservoir (16) ein Wirkstoff in fester, flüssiger oder pulverförmiger Form bevorratet ist.

6. Intradermales Wirkstoffabgabesystem nach einem der Ansprüche 1-5, **dadurch gekennzeichnet, dass** das flüssigen Wirkstoff enthaltende Wirkstoffreservoir (16) und die Entnahmeeinrichtung (10) mit einem Einlass (20) der Fördereinrichtung (14), insbesondere einem Pumpeneinlass, verbunden sind.

7. Intradermales Wirkstoffabgabesystem nach Anspruch 6, **dadurch gekennzeichnet, dass** das Wirkstoffreservoir (16) und die Entnahmeeinrichtung (10) derart mit dem Einlass der Fördereinrichtung (14), insbesondere dem Pumpeneinlass, verbunden sind, dass der Wirkstoff und die Initialflüssigkeit gleichzeitig angesaugt werden.

8. Intradermales Wirkstoffabgabesystem nach einem der Ansprüche 1-5, **dadurch gekennzeichnet, dass** das Wirkstoffreservoir (16) mit einem Auslass (22), der Fördereinrichtung, insbesondere einem Pumpenauslass, verbunden ist.

9. Intradermales Wirkstoffabgabesystem nach einem der Ansprüche 1-8, **dadurch gekennzeichnet, dass** die Fördereinrichtung zwei Pumpen (14,40) aufweist und das Wirkstoffreservoir (16) zwischen den beiden Pumpen (14,40) angeordnet ist.

10. Intradermales Wirkstoffabgabesystem nach einem der Ansprüche 1-9. **dadurch gekennzeichnet, dass** die Abgabeeinrichtung (26) Mikronadeln, insbesondere ein Mikroarray, mit mindestens 4 Nadeln/cm², insbesondere mindestens 50 Nadeln/cm² und besonders bevorzugt mindestens 100 Nadeln/cm², aufweist.

11. Intradermales Wirkstoffabgabesystem nach einem der Ansprüche 1-10, **dadurch gekennzeichnet, dass** die Abgabeeinrichtung mit dem Auslass (22,44) der Fördereinrichtung (14), insbesondere dem Pumpenauslass, verbunden ist.

12. Intradermales Wirkstoffabgabesystem nach einem der Ansprüche 1-11, **dadurch gekennzeichnet, dass** das Wirkstoffreservoir (16) in die Abgabeeinrichtung (26) integriert ist, wobei der Wirkstoff vorzugsweise in den Nadeln (30) der Abgabeeinrichtung (26) vorgesehen ist.

13. Intradermales Wirkstoffabgabesystem nach einem der Ansprüche 1-12, **gekennzeichnet durch** eine Mischkammer zum Mischen von Interstitial-Flüssigkeit und Wirkstoff.

14. Intradermales Wirkstoffabgabesystem nach Anspruch 13, **dadurch gekennzeichnet, dass** das Wirkstoffreservoir (16) die Mischkammer ausbildet.

15. Intradermales Wirkstoffabgabesystem nach Anspruch 13 oder 14, **dadurch gekennzeichnet, dass** die Mischkammer in die Fördereinrichtung (14) integriert ist.

## Claims

1. An intradermal active ingredient delivery system comprising
an extraction device (10) for extracting interstitial liquid,
a conveying device (14) fluidically connected to the extraction device (10) for conveying liquid,
an active ingredient reservoir (16) fluidically connected to the conveying device, and
a delivery device (26) fluidically connected to the conveying device (14) for intradermal delivery of the interstitial liquid comprising an active ingredient.

2. The intradermal active ingredient delivery system according to claim 1, **characterized in that** the extraction device (10) comprises microneedles (30), in particular a microarray having at least 4 needles/cm², in particular at least 50 needles/cm², and particularly preferred at least 100 needles/cm².

3. The intradermal active ingredient delivery system according to claim 2, **characterized in that** the extraction device (10) comprises two microarrays each having in particular at least 4 needles/cm², in particular at least 50 needles/cm², and particularly preferred at least 100 needles/cm².

4. The intradermal active ingredient delivery system according to any one of claims 1-3, **characterized in that** the conveying device (16) comprises at least one pump.

5. The intradermal active ingredient delivery system according to any one of claims 1-4, **characterized in that** an active ingredient in solid, liquid or powder form is stored in the active ingredient reservoir (16).

6. The intradermal active ingredient delivery system according to any one of claims 1-5, **characterized in that** the active ingredient reservoir (16) containing the liquid active ingredient and the extraction device (10) are connected to an inlet (20) of the conveying device (14), in particular to a pump inlet.

7. The intradermal active ingredient delivery system according to claim 6, **characterized in that** the active ingredient reservoir (16) and the extraction device (10) are connected to the inlet of the conveying device (14), in particular to the pump inlet such that the active ingredient and the initial liquid are drawn in simultaneously.

8. The intradermal active ingredient delivery system according to any one of claims 1-5, **characterized in that** the active ingredient reservoir (16) is connected to an outlet (22) of the conveying device, in particular to a pump outlet.

9. The intradermal active ingredient delivery system according to any one of claims 1-8, **characterized in that** the conveying device comprises two pumps (14, 40), and that the active ingredient reservoir (16) is arranged between the two pumps (14, 40).

10. The intradermal active ingredient delivery system according to any one of claims 1-9, **characterized in that** the delivery device (26) comprises microneedles, in particular a microarray having at least 4 needles/cm², in particular at least 50 needles/cm², and particularly preferred at least 100 needles/cm².

11. The intradermal active ingredient delivery system according to any one of claims 1-10, **characterized in that** the delivery device is connected to the outlet (22, 44) of the conveying device (14), in particular to the pump outlet.

12. The intradermal active ingredient delivery system according to any one of claims 1-11, **characterized in that** the active ingredient reservoir (16) is integrated into the delivery device (26), wherein the active ingredient is preferably provided in the needles (30) of the delivery device (26).

13. The intradermal active ingredient delivery system according to any one of claims 1-12, **characterized by** a mixing chamber for mixing interstitial liquid and active ingredient.

14. The intradermal active ingredient delivery system according to claim 13, **characterized in that** the active ingredient reservoir (16) forms the mixing chamber.

15. The intradermal active ingredient delivery system according to claim 13 or 14, **characterized in that** the mixing chamber is integrated into the conveying device (14).

## Revendications

1. Système d'administration de principe actif par voie intradermique, doté
d'un dispositif d'extraction (10) destiné à extraire du liquide interstitiel,
d'un dispositif de transfert (14) relié fluidiquement au dispositif d'extraction (10) destiné à transférer du liquide,
d'un réservoir de principe actif (16) relié fluidiquement au dispositif de transfert et
d'un dispositif d'administration (26) relié fluidiquement au dispositif de transfert (14) destiné à l'administration intradermique du liquide interstitiel comportant du principe actif.

2. Système d'administration de principe actif par voie intradermique selon la revendication 1, **caractérisé en ce que** le dispositif d'extraction (10) comporte des micro-aiguilles (30), en particulier un réseau de micro-aiguilles doté d'au moins 4 aiguilles/cm², en particulier d'au moins 50 aiguilles/cm² et tout particulièrement d'au moins 100 aiguilles/cm².

3. Système d'administration de principe actif par voie intradermique selon la revendication 2, **caractérisé en ce que** le dispositif d'extraction (10) comporte deux réseaux de micro-aiguilles dotés respectivement d'au moins 4 aiguilles/cm², en particulier d'au moins 50 aiguilles/cm² et tout particulièrement d'au moins 100 aiguilles/cm².

4. Système d'administration de principe actif par voie intradermique selon l'une des revendications 1-3, **caractérisé en ce que** le dispositif de transfert (16) comporte au moins une pompe.

5. Système d'administration de principe actif par voie intradermique selon l'une des revendications 1-4, **caractérisé en ce qu'**un principe actif est stocké sous forme solide, liquide ou pulvérulente dans le réservoir de principe actif (16).

6. Système d'administration de principe actif par voie intradermique selon l'une des revendications 1-5, **caractérisé en ce que** le réservoir de principe actif (16) contenant du principe actif liquide et le dispositif d'extraction (10) sont reliés à une entrée (20) du dispositif de transfert (14), en particulier une entrée de pompe.

7. Système d'administration de principe actif par voie intradermique selon la revendication 6, **caractérisé en ce que** le réservoir de principe actif (16) et le dispositif d'extraction (10) sont reliés à l'entrée du dispositif de transfert (14), en particulier l'entrée de pompe, de telle sorte que le principe actif et le liquide initial sont aspirés simultanément.

8. Système d'administration de principe actif par voie intradermique selon l'une des revendications 1-5, **caractérisé en ce que** le réservoir de principe actif (16) est relié à une sortie (22) du dispositif de transfert, en particulier une sortie de pompe.

9. Système d'administration de principe actif par voie intradermique selon l'une des revendications 1-8, **caractérisé en ce que** le dispositif de transfert comporte au moins deux pompes (14, 40) et le réservoir de principe actif (16) est disposé entre les deux pompes (14, 40).

10. Système d'administration de principe actif par voie intradermique selon l'une des revendications 1-9, **caractérisé en ce que** le dispositif d'administration (26) comporte des micro-aiguilles, en particulier un réseau de micro-aiguilles doté d'au moins 4 aiguilles/cm², en particulier d'au moins 50 aiguilles/cm² et tout particulièrement d'au moins 100 aiguilles/cm².

11. Système d'administration de principe actif par voie intradermique selon l'une des revendications 1-10, **caractérisé en ce que** le dispositif d'administration est relié à la sortie (22, 24) du dispositif de transfert (14), en particulier la sortie de pompe.

12. Système d'administration de principe actif par voie intradermique selon l'une des revendications 1-11, **caractérisé en ce que** le réservoir de principe actif (16) est intégré dans le dispositif d'administration (26), dans lequel le principe actif est de préférence prévu dans les aiguilles (30) du dispositif d'administration (26) .

13. Système d'administration de principe actif par voie intradermique selon l'une des revendications 1-12, **caractérisé par** une chambre mélangeuse destiné à mélanger le liquide interstitiel et le principe actif.

14. Système d'administration de principe actif par voie intradermique selon la revendication 13, **caractérisé en ce que** le réservoir de principe actif (16) réalise la chambre mélangeuse.

15. Système d'administration de principe actif par voie intradermique selon la revendication 13 ou 14, **caractérisé en ce que** la chambre mélangeuse est intégrée dans le dispositif de transfert (14) .
